# NEUE EUROPÄISCHE PATENTSCHRIFT

(11) **EP 2 666 491 B2**
(45) Veröffentlichungstag und Bekanntmachung des Hinweises auf die Entscheidung über den Einspruch: **12.08.2020**
(45) Hinweis auf die Patenterteilung: 26.04.2017
(21) Anmeldenummer: 12168954.1
(22) Anmeldetag: 22.05.2012
(51) Int. Cl.: A61M 1/16, A61M 1/34

(54) **Hämodialysegerät**
Haemodialysis device
Appareil d'hémodialyse

(43) Veröffentlichungstag der Anmeldung: 27.11.2013
(73) Patentinhaber: D.Med Consulting GmbH, 20359 Hamburg (DE)
(72) Erfinder: Biermann, Frank, 22455 Hamburg (DE); Breuch, Gerd, 53844 Troisdorf (DE); Yanagimoto, Yoji, 3080 Tervuren (Moorsel) (BE)
(74) Vertreter: terpatent Patentanwälte ter Smitten Eberlein-Van Hoof Rütten Daubert

(56) Entgegenhaltungen:
- EP-A1- 2 116 269
- WO-A1-00/06217
- WO-A1-98/52628
- WO-A1-2010/121750
- WO-A1-2010/130202
- DE-A1- 3 444 671
- DE-A1- 4 239 937
- DE-A1- 10 224 750
- DE-A1- 10 245 619
- DE-C1- 3 743 272
- DE-C1- 10 049 900
- JP-A- 2011 200 407
- US-A- 4 702 829
- US-A1- 2004 089 594
- US-A1- 2005 131 331
- US-A1- 2011 163 030
- "BASISWISSEN VERDRÄNGERPUMPEN", Hydraulische Fluidenergiemaschinen, page 307, Retrieved from the Internet: URL:http://www.gunt.de/images/download/pos itive-displacement-pumps_german.pdf

## Beschreibung

0001 Die Erfindung bezieht sich auf ein Hämodialysegerät, das eine dauerhaft benutzte und desinfizierbare Internfluidik und eine für den Einmalgebrauch ausgelegte Wegwerf- Externfluidik aufweist.

0002 Bei den bekannten Hämodialysegeräten zählt zur Internfluidik insbesondere die Dialysatfluidik einschließlich einer Bilanzierpumpe, die aus einer Dialysatpumpe und einer mit dieser zwangsweise gekoppelten Abfallpumpe gebildet ist. Die Kopplung kann mechanisch oder auf andere Weise ausgebildet sein, beispielsweise in Form einer programmierten zwangsweisen Kopplung. Die Dialysatpumpe pumpt Dialysat von einer Dialysatquelle zu der Dialysatkammer eines Dialysators. Die Abfallpumpe pumpt das Dialysat von dem Dialysator zu einem Abfallziel, beispielsweise einem Abfallbehälter. Zu der als Schlauchset ausgebildeten Wegwerf-Externfluidik für den Einmalgebrauch zählt insbesondere die gesamte Blutseite einschließlich der arteriellen Leitung und der venösen Leitung, die beide an eine Blutkammer des Dialysators angeschlossen sind.

0003 Ferner gehört zu dem Externfluidik- Schlauchset eine Substituatleitung, die über eine entsprechende Gerätekupplung mit der Dialysequelle verbunden ist und eine Verbindung zu einem Substituatzugang an einer der blutseitigen Leitungen herstellt. Für die Flüssigkeitsförderung in den Leitungen der Externfluidik sind externe Pumpen vorgesehen, insbesondere eine Blutpumpe und eine Substituatpumpe für die Substituatleitung. Die externen Pumpen sind als peristaltische Schlauchpumpen ausgebildet, wobei die Pumpenmimik von außen zugänglich auf der Außenseite des Hämodialysegeräts vorgesehen ist. Schlauchpumpen sind bezüglich der Flussrate prinzipbedingt mit einer Ungenauigkeit in der Größenordnung von 10 % behaftet. Um ein einfaches und fehlerfreies Applizieren des Externfluidik- Schlauchsets in die externe Pumpenmimik zu gewährleisten, ist das externe Schlauchset in diesen Bereichen mit aufwändigen Pumpenadaptern versehen.

EP 2 116 269 A1 und JP 2011-200407 A offenbaren Hämodialysegeräte mit Substituatpumpen, die als externe peristaltische Schlauchpumpen ausgebildet sind.

US 2004/0089594 A1 und US 4702829 offenbaren Hämodialysegeräte, die ebenfalls einen Substituatpfad mit einer Substituatpumpe aufweisen.

Aufgabe der Erfindung ist es demgegenüber, ein Hämodialysegerät mit einer einfach handhabbaren Externfluidik zu schaffen.

Diese Aufgabe wird erfindungsgemäß mit einem Hämodialysegerät mit den Merkmalen des Anspruchs 1 gelöst.

Bei dem erfindungsgemäßen Hämodialysegerät ist die Substituatpumpe in der Internfluidik angeordnet, d.h., die Substituatpumpe ist nicht als externe Pumpe ausgebildet, deren flüssigkeitsführender Teil austauschbar ist. Sie muss daher auch nicht zwangsläufig als relativ ungenaue peristaltische Schlauchpumpe ausgebildet sein, sondern kann beispielsweise als eine relativ genaue Kolbenpumpe ausgebildet sein. Da die interne Substituatpumpe keine flüssigkeitsführenden Teile aufweist, die austauschbar sind, ist die Substituatpumpe desinfizierbar ausgebildet.

Es ist eine fluidische Substituat- Kupplung vorgesehen, über die die Substituatpumpe über eine weiterführende Substituatleitung der Externfluidik fluidisch mit dem Substituatzugang verbunden ist. Da die Substituatpumpe in der Internfluidik angeordnet ist, ist das Applizieren des Externfluidik-Schlauchsets erheblich vereinfacht. Ferner ist das externe Externfluidik-Schlauchset durch den Wegfall eines Pumpenadapters erheblich einfacher und preiswerter.

Die Substituatpumpe ist als eine nicht-peristaltische Verdrängerpumpe ausgebildet. Bevorzugt ist die Substituatpumpe als eine keramische Kolbenpumpe ausgebildet. Eine Verdrängerpumpe bietet eine volumetrische Genauigkeit im einstelligen Prozentbereich. Die Ausbildung als keramische Pumpe stellt eine lange Lebensdauer, eine hohe Langzeitgenauigkeit sowie eine problemlose Desinfizierbarkeit sicher.

0004 Vorzugsweise ist die Zuleitung der Substituatpumpe fluidisch zwischen der Dialysatpumpe und dem Dialysator innerhalb der Internfluidik angeschlossen. Das Dialysat für die Substituatpumpe wird also, in Flussrichtung gesehen, hinter der Dialysatpumpe abgezweigt. Da die Dialysatpumpe und die Abfallpumpe zusammen als Bilanzierpumpe ausgebildet sind, wird den blutseitigen Leitungen über die Dialysator-Membran des Dialysators stets so viel Flüssigkeit entzogen, wie der betreffenden blutseitigen Leitung über die Substituatleitung zugeführt wird. Die Flüssigkeitsmenge, die dem blutseitigen Leitungen über den Substituatzugang zugeführt wird, wird durch die Dialysator-Membran hindurch von der Blutkammer zur Dialysatkammer ultrafiltriert.

0005 Alternativ kann die Zuleitung zu der Substituatpumpe fluidisch zwischen der Dialysatquelle und der Dialysatpumpe angeschlossen sein, wobei dann fluidisch parallel zu der Abfallpumpe eine im Parallelstrom betreibbare Ultrafiltrationspumpe vorgesehen ist. Da das Substituat in diesem Fall außerhalb des Bilanzkreises abgezweigt wird, ist für eine volumenneutrale Substitution eine weitere Pumpe, nämlich bevorzugt die Ultrafiltrationspumpe, im dialysatseitigen Abfallzweig vorgesehen, die parallel zur Abfallpumpe und in derselben Richtung wie die Abfallpumpe betrieben werden kann. Die Pumprate der Ultrafiltrationspumpe sollte dabei stets der Pumprate der Substituatpumpe entsprechen, wenn eine volumenneutrale Substitution gewünscht ist. Der Substituatzugang kann bei dieser Konstellation auch in der Internfluidik angeordnet sein, so dass eine Substituat-Kupplung entfällt.

0006 Gemäß einer bevorzugten Ausgestaltung ist der Substituatzugang zu der blutseitigen Leitung stromaufwärts des Dialysators vorgesehen, so dass der Substituatzugang in die arterielle Leitung mündet. Alternativ kann der Substituatzugang jedoch auch stromabwärts des Dialysators vorgesehen sein, also in die venöse Leitung hinter dem Dialysator münden. Gemäß einer weiteren Ausführungsform kann der Substituatzugang auf der Blutseite unmittelbar an der Blutkammer des Dialysators angeordnet sein.

0007 Gemäß einer weiteren alternativen oder ergänzenden Ausführungsform ist eine zweite und mit der Substituatpumpe verbundene Substituatkupplung sowie eine Ventilanordnung zwischen der Substituatpumpe und den beiden Substituatkupplungen vorgesehen. Die beiden Substituatkupplungen sind mit zwei verschiedenen externfluidischen Substituatzugängen verbunden.

0008 Mit der Ventilanordnung kann zwischen den beiden verschiedenen Substituatzugängen alternativ geschaltet werden, beispielsweise zwischen einem Substituatzugang stromabwärts und einem Substituatzugang stromaufwärts des Dialysators.

0009 Vorzugsweise ist der Substituatpumpe fluidisch in Reihe ein Durchfluss-Sensor zugeordnet. Der Durchfluss-Sensor ist insbesondere fluidisch vor der Substituatkupplung angeordnet, beispielsweise unmittelbar stromabwärts oder stromaufwärts der Substituatpumpe. Hierdurch kann der Substituatpumpen-Durchfluss exakt ermittelt werden. Dies ist insbesondere dann erforderlich, wenn die Zuleitung der Substituatpumpe fluidisch stromaufwärts der Dialysatpumpe angeschlossen ist. Die Ultrafiltrationspumpe kann dann mit einer Pumprate betrieben werden, die exakt der durch den Durchfluss-Sensor gemessenen Flussrate entspricht.

0010 Im Folgenden werden mehrere Ausführungsbeispiele der Erfindung anhand der Zeichnungen näher erläutert.

0011 Es zeigen:
Figur 1 eine schematische Darstellung eines Hämodialysegeräts in einer ersten Ausführungsform mit einer Substituatpumpen-Zuleitung stromabwärts der Dialysatpumpe und einem Substituatzugang stromaufwärts des Dialysators,
Figur 2 eine schematische Darstellung eines Hämodialysegeräts in einer zweiten Ausführungsform mit einem Substituatzugang stromabwärts des Dialysators,
Figur 3 eine schematische Darstellung eines Hämodialysegeräts in einer dritten Ausführungsform mit einem Substituatzugang an der Blutkammer des Dialysators,
Figur 4 eine schematische Darstellung eines Hämodialysegeräts einer vierten Ausführungsform mit zwei verschiedenen Substituatzugängen stromaufwärts und stromabwärts des Dialysators, und
Figur 5 eine schematische Darstellung eines Hämodialysegeräts in einer fünften Ausführungsform, die nicht in den Schutzbereich dieses Patents fällt, mit einer Substituatpumpen- Zuleitung stromaufwärts der Dialysatpumpe und einer Ultrafiltrationspumpe parallel zu der Abfallpumpe, wobei der Substituatzugang in der Internfluidik angeordnet ist.

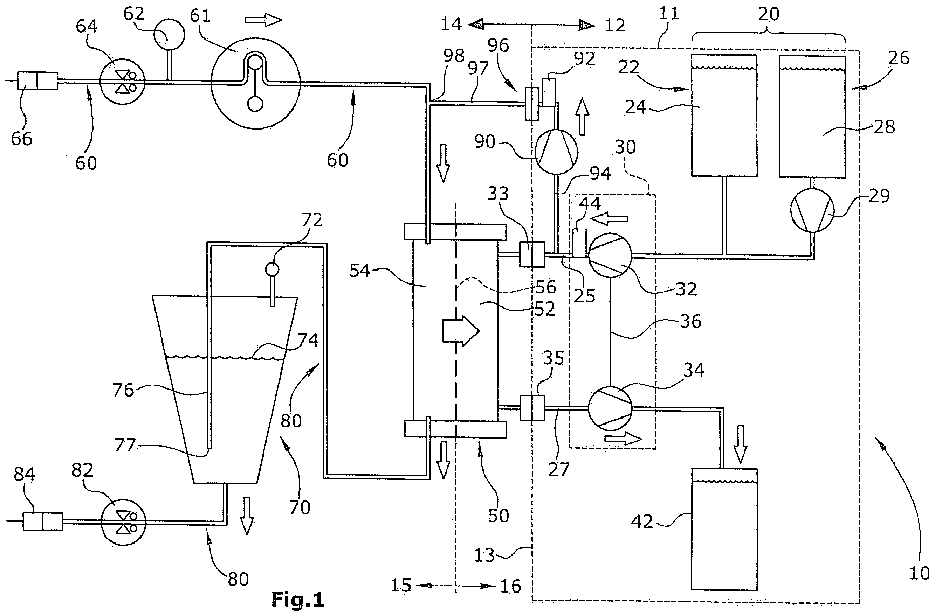

0012 In den Figuren 1-5 ist schematisch jeweils ein Hämodialysegerät 10 dargestellt, das funktional in eine Dialysatseite 15 und eine Blutseite 16 unterteilt werden kann. Die Grenze zwischen der Dialysatseite 15 und der Blutseite 16 wird von einer Dialyse-Membran 56 in einem Dialysator 50 gebildet. Die Dialyse-Membran 56 trennt in dem Dialysator 50 eine Dialysatkammer 52 von einer Blutkammer 54. Strukturell ist das Hämodialysegerät 10 unterteilt in eine wiederholt einsetzbare und desinfizierbare Internfluidik 12 innerhalb eines Gerätegehäuses 11 einerseits und eine austauschbare Wegwerf- Externfluidik 14 außerhalb des Gerätegehäuses 11, die nicht desinfizierbar sein muss und nach jeder Dialyse-Behandlung ausgetauscht wird.

0013 Die Dialysatseite 12 weist eine Dialysatquelle 20 auf, die Dialysat für die Dialyse zur Verfügung stellt. Das Dialysat dient auch als Substituat, so dass die Dialysatquelle 20 auch als Substituatquelle bezeichnet werden kann. Die Dialysatquelle 20 wird von einem Dialysewasser-Tank 22 mit Dialysewasser 24 und einem Additiv-Tank 26 mit einem Dialysat- Additiv 28 gebildet. Dem Additiv-Tank 26 ist eine Additiv-Pumpe 29 nachgeordnet, die ein Additiv 28 bei Bedarf wohldosiert dem Dialysewasser-Strom zuführt. Es können auch mehrere Additiv-Tanks vorgesehen sein, wobei als Additive insbesondere Elektrolyte und Puffersubstanzen vorgesehen sind. Das Dialysewasser kann, alternativ zu dem Dialysewasser-Tank 22, auch von einer (nicht dargestellten) Wasseraufbereitungs-Vorrichtung zur Verfügung gestellt werden, in der aus Trinkwasser aus dem öffentlichen Leitungsnetz das Dialysewasser hergestellt wird.

0014 Das Dialysat bzw. die identische Substituatflüssigkeit aus der Dialysatquelle 20 wird durch eine Dialysatpumpe 32 über eine Dialysatleitung 25 in die Dialysatkammer 52 des Dialysators 50 gepumpt. Von der Dialysatkammer 52 aus verläuft eine Leitung zu einer Abfallpumpe 34, die das Dialysat aus der Dialysatkammer 52 in einen Abfalltank 42 pumpt, der das Abfallziel darstellt, und in dem das verbrauchte Dialysat während der Dialysebehandlung gespeichert wird.

0015 Die Dialysatpumpe 32 und die Abfallpumpe 34 sind mechanisch durch eine mechanische Verbindung 36 miteinander verbunden, so dass die Pumpraten der Dialysatpumpe 32 und der Abfallpumpe 34 stets absolut identisch sind. Auf diese Weise bilden die Dialysatpumpe 32 und die Abfallpumpe 34 eine sogenannte Bilanzierpumpe 30. Die mechanische Verbindung 36 kann beispielsweise dadurch realisiert sein, dass die Bilanzierpumpe 30 als Membranpumpe ausgebildet ist, wobei die eine Seite der Pumpenmembran die Pumpkammer der Dialysatpumpe 32 und die andere Seite der Pumpenmembran die Pumpkammer der Abfallpumpe 34 begrenzt.

0016 Auf der Blutseite 15 der Dialysator- Membran 56 befindet sich die Blutkammer 54 des Dialysators 50, in die eine arterielle Leitung 60 und eine venöse Leitung 80 münden. Im Verlauf der arteriellen Leitung 60 ist eine Blutpumpe 61 angeordnet, die als peristaltische Schlauchpumpe ausgebildet ist, wobei die peristaltische Pumpmimik an dem Gerätegehäuse 11 angeordnet ist, in die die als flexibler Schlauch ausgebildete Leitung 60 eingelegt ist. Die Pumprate der Blutpumpe 61 wird von einer nicht dargestellten Gerätesteuerung gesteuert. Im Verlauf der arteriellen Leitung 60 ist ein arterieller Drucksensor 62 angeordnet, durch den der statische Fluiddruck in der arteriellen Leitung detektiert wird. Auch der arterielle Drucksensor 62 ist mit der Gerätesteuerung verbunden. Stromaufwärts des arteriellen Drucksensors 62 ist eine arterielle Schlauchklemme 64 angeordnet, die die als flexibler Schlauch ausgebildete arterielle Leitung 60 schließen oder öffnen kann. Am Ende der arteriellen Leitung 60 ist eine arterielle Kanüle 66 angeordnet, die eine Nadel aufweist, mit der die arterielle Leitung 60 an ein Blutgefäß eines Patienten appliziert werden kann, aus dem die Blutpumpe 61 zur Durchführung der eigentlichen Blutreinigung Patientenblut in die arterielle Leitung 60 absaugt.

0017 Im Verlauf der venösen Leitung 80 ist eine Luftfalle 70 angeordnet, die ein Tauchrohr 76 mit einer nach unten öffnenden Rohröffnung 77 aufweist. Die Rohröffnung 77 liegt weit unterhalb eines Flüssigkeitspegels 74, dessen Höhe über einen separaten obenliegenden Gasanschluss (nicht dargestellt) und eine daran angeschlossene Pegelregelpumpe (nicht dargestellt) auf einen Sollpegel geregelt wird. Durch die Pegelregelpumpe wird sichergestellt, dass die Rohröffnung 77 stets unterhalb des Flüssigkeitspegels 74 liegt. An der Luftfalle 70 ist ferner ein venöser Drucksensor 72 angeordnet, der den Fluiddruck in der venösen Leitung 80 detektiert. Der Drucksensor 72 kann auch an den Gasanschluss für die Pegelregelpumpe angeschlossen sein.

0018 Im weiteren Verlauf der als flexibler Schlauch ausgebildeten venösen Leitung 80 ist eine venöse Leitungsklemme 82 vorgesehen, die mit der Gerätesteuerung verbunden ist und ein schaltbares Ventil darstellt, das die venöse Leitung 80 schließt oder öffnet. An dem freien Ende der venösen Leitung 80 ist eine venöse Kanüle 84 vorgesehen, die eine Nadel aufweist, so dass die venöse Kanüle 84 zu Beginn der eigentlichen Blutreinigung an ein entsprechendes venöses Blutgefäß des Patienten appliziert werden kann.

0019 In dem in der Figur 1 dargestellten Ausführungsbeispiel des Hämodialysegeräts 10 ist innerhalb des Gerätegehäuses 11 eine Substituatpumpe 90 angeordnet, die Dialysat bzw. Substituat zu einem externen Substituatzugang 98 pumpt, der an der arteriellen Leitung 60 stromaufwärts der Dialysator- Blutkammer 54 angeordnet ist. Hinter der Dialysatpumpe 32 ist ein erster Dialysatfilter 44 angeordnet, der zusammen mit einem zweiten Dialysatfilter 44 die Sterilität des austretenden Dialysats bzw. Substituats redundant sicherstellt. Der zweite Dialysatfilter 44 kann, je nach Ausführungsbeispiel, als ein integrierter oder separater Filter beispielsweise ebenfalls direkt hinter der Dialysatpumpe 32 angeordnet sein. Grundsätzlich kann der oder können die Dialysatfilter 44 fluidisch auch hinter der betreffenden Substituatpumpe angeordnet sein. Die Zuleitung 94 der Substituatpumpe 90 ist fluidisch von der Dialysatleitung 25 zwischen der Dialysatpumpe 32 und dem Dialysator 50 innerhalb des Gerätegehäuses 11 abgezweigt. Stromabwärts der Substituatpumpe 90 ist ein Durchfluss-Sensor 92 angeordnet, der den Substituat- Durchfluss quantitativ erfasst. Die Substituatpumpe 90, ihre Zuleitung 94 und der Durchfluss-Sensor 92 sind innerhalb des Gerätegehäuses 11 angeordnet, gehören also zur Internfluidik 12. An der Bedienungsseite 13 des Hämodialysegeräts 10 ist eine Substituat- Kupplung 96 angeordnet, die bezüglich der SubstituatLeitung die Schnittstelle zwischen der Internfluidik 12 und der Externfluidik 14 darstellt. An die Substituatkupplung 96 ist die externfluidische Substituatleitung 97 angeschlossen, die zu dem Substituatzugang 98 führt.

0020 Auch für die von der Dialysatpumpe 32 zu der Dialysatkammer 52 des Dialysators 50 führende Dialysatleitung 25 und die von der Dialysatkammer 52 zu der Abfallpumpe 34 führende Abfallleitung 27 sind entsprechend eine Dialysatkupplung 33 und eine Abfallkupplung 35 an der Geräte- Bedienungsseite 13 vorgesehen. Die vorgenannten Leitungskupplungen 96,33,35 stellen jeweils die fluidische Schnittstelle zwischen der Internfluidik 12 und der Externfluidik 14 dar.

0021 Im Substituat-Betrieb läuft die Bilanzierpumpe 30 kontinuierlich, so dass kontinuierlich Dialysat bzw. Substituat von der Dialysatquelle 20 durch die Dialysatpumpe 32 gefördert wird. Gleichzeitig fördert die Substituatpumpe 90 mit einer Pumprate, die unterhalb der Pumprate der Dialysatpumpe 32 liegt, Substituat zu dem Substituatzugang 98. Zum Bilanzausgleich wird in dem Dialysator mit einer Ultrafiltrationsrate, die der Pumprate der Substituatpumpe 90 zuzüglich der Ultrafiltrationsrate des Patienten entspricht, durch die Dialysator- Membran 56 hindurch zur Dialysatseite 16 hin ultrafiltriert.

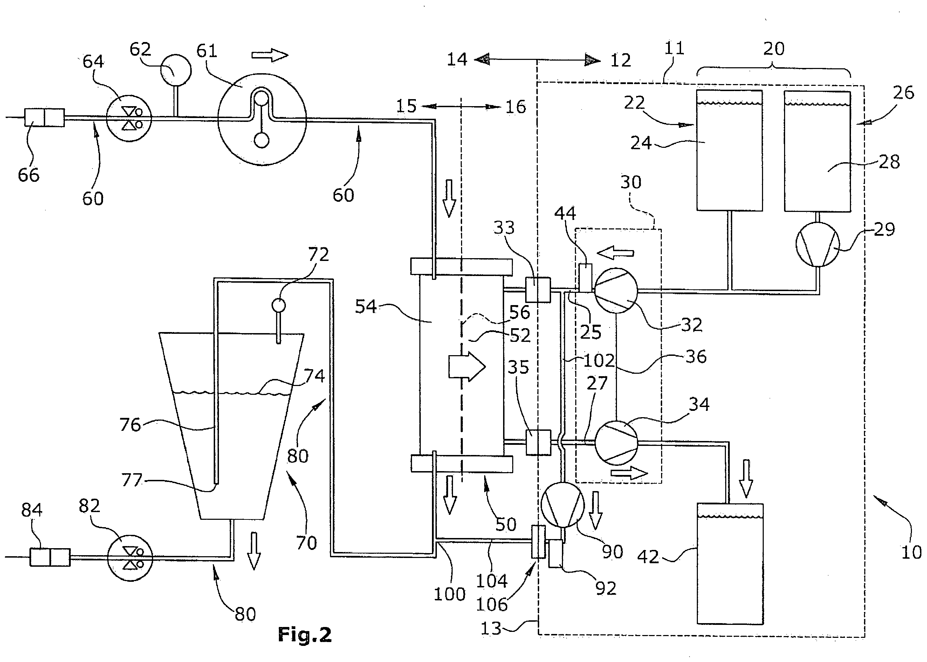

0022 In der Figur 2 ist ein anderes Ausführungsbeispiel eines Hämodialysegeräts 10 dargestellt, wobei der wesentliche Unterschied zu dem Hämodialysegerät 10 der Figur 1 darin besteht, dass der Substituatzugang 100 stromabwärts des Dialysators 50 bzw. der Dialysator- Blutkammer 54 angeordnet ist. Auch in diesem Ausführungsbeispiel zweigt die Substituatleitung 102 stromabwärts der Dialysatpumpe 32 von der Dialysatleitung 25 ab, und ist über eine Substituatkupplung 106 mit der externfluidischen Substituatleitung 104 verbunden, die zu dem Substituatzugang 100 führt.

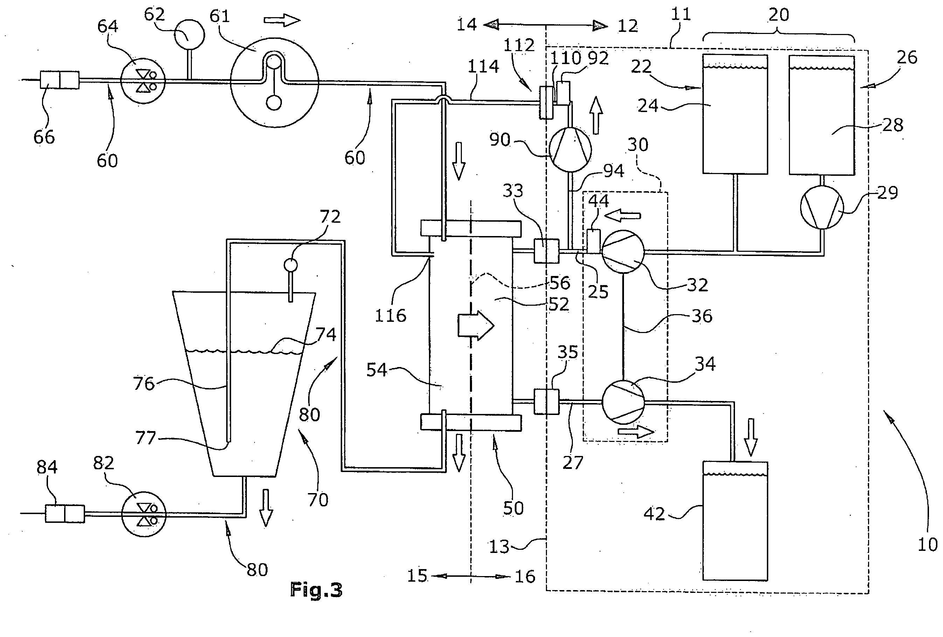

0023 In dem in der Figur 3 dargestellten dritten Ausführungsbeispiel eines Hämodialysegeräts 10 ist der Substituatzugang 116 direkt an der Blutkammer 54 des Dialysators 50 angeordnet. Die externfluidische Substituatleitung 114 ist über eine Substituatkupplung 112 mit der internfluidischen Substituatleitung 110 verbunden.

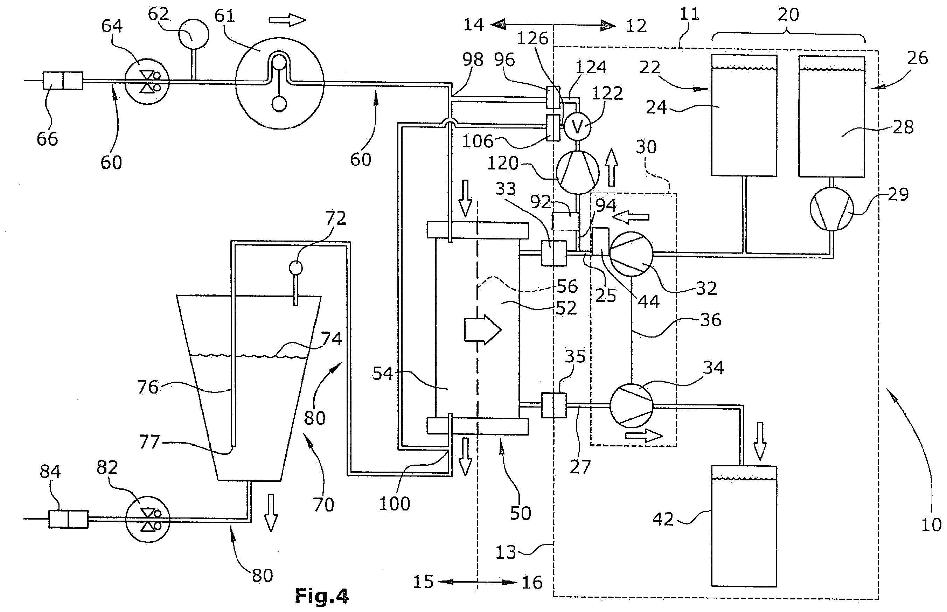

0024 In dem in der Figur 4 dargestellten vierten Ausführungsbeispiel eines Hämodialysegeräts 10 ist internfluidisch und innerhalb des Gerätegehäuses 11 stromabwärts der Substituatpumpe 120 eine Umschalt- Ventilanordnung 122 vorgesehen, durch die das von der Substituatpumpe 120 geförderte Substituat über eine entsprechende Substituatkupplung 96,106 an einen Substituatzugang 98 stromaufwärts des Dialysators 50 und/oder an einen Substituatzugang 100 stromabwärts des Dialysators 50 geleitet werden kann. Alternativ oder ergänzend kann die Ventilanordnung auch an einen Substituatzugang an dem Dialysator angeschlossen sein. Von der Ventilanordnung 122 führt eine Substituatleitung 124 zu der Substituatkupplung 96 für den stromaufwärtigen Substituatzugang 98 und eine andere Substituatleitung 126 zu der anderen Substituatkupplung 106 zu dem stromabwärtigen Substituatzugang 100. Die Ventilanordnung 122 ist an eine Gerätesteuerung (nicht dargestellt) angeschlossen.

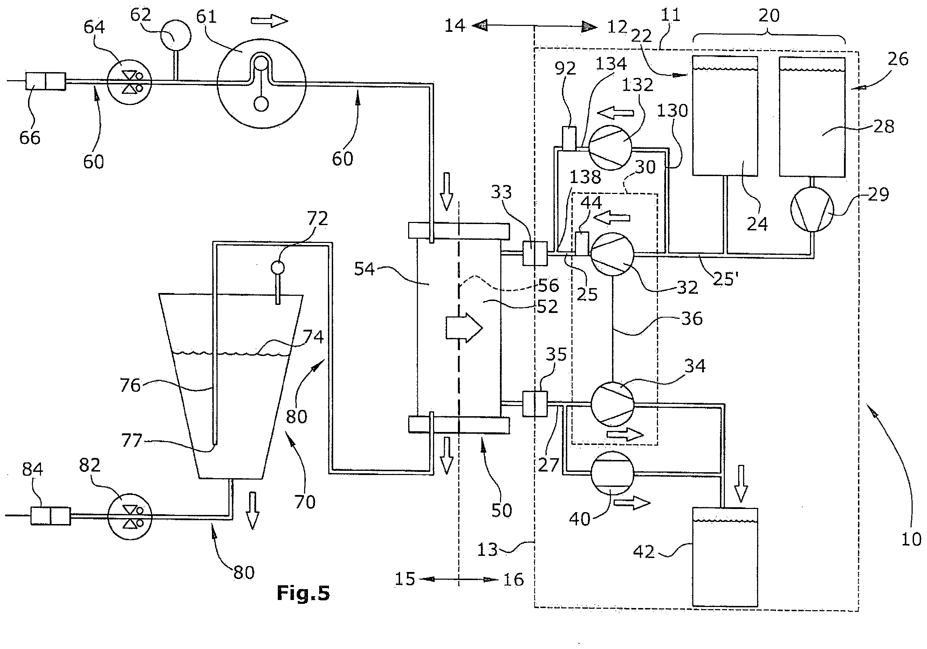

0025 In dem in der Figur 5 dargestellten fünften Ausführungsbeispiel eines Hämodialysegeräts 10 ist die Zuleitung 130 der Substituatpumpe 132 fluidisch an der Substituatleitung 25' zwischen der Dialysatquelle 20 und der Dialysatpumpe 32 angeschlossen, zweigt also stromaufwärts der Dialysatpumpe 32 von der Dialysatleitung 25' ab. Der Substituatzugang 138 ist, im Unterschied zu den Ausführungen gemäß den Fig. 1-4, hier in der Internfluidik 12 vorgesehen, nämlich an der internfluidischen Dialysatleitung 25. Fluidisch parallel zu der Abfallpumpe 34 ist eine Ultrafiltrationspumpe 40 vorgesehen, die im Substituatbetrieb im Parallelstrom zu der Abfallpumpe 34 betrieben wird. Hierbei wird die Ultrafiltrationspumpe 40 ggf. mit derselben Pumprate betrieben, wie die der Substituatpumpe 132, zuzüglich der Ultrafiltrationsrate des Patienten.

## Patentansprüche

1. Hämodialysegerät (10) mit einer dauerhaften und desinfizierbaren Internfluidik (12) innerhalb eines Gerätegehäuses (11) und einer austauschbaren Wegwerf- Externfluidik (14) außerhalb des Gerätegehäuses (11), wobei
die Externfluidik (14) alle blutseitigen Leitungen (60,80) und einen Dialysator (50) aufweist, und
die Internfluidik (12)
eine Bilanzierpumpe (30), die aus einer Dialysatpumpe (32) und einer mit dieser gekoppelten Abfallpumpe (34) gebildet ist, wobei die Dialysatpumpe (32) Dialysat von einer Dialysatquelle (20) zu dem Dialysator (50) und die Abfallpumpe (34) Dialysat von dem Dialysator (50) zu einem Abfallziel pumpt, und
eine separate desinfizierbare Substituatpumpe (90; 120; 132), die Dialysat von der Dialysatquelle (20) zu der Externfluidik (14) pumpt,
aufweist,
wobei die Substituatpumpe (90;120;132) eine nicht- peristaltische Verdrängerpumpe mit einer volumetrischen Genauigkeit im einstelligen Prozentbereich ist,
wobei die Externfluidik einen separaten Substituatzugang (98;100;116) zur Blutseite (15) der Externfluidik (14) aufweist und eine Substituat-Kupplung (96;106;112) vorgesehen ist, über die die Substituatpumpe (90;120;132) fluidisch mit dem Substituatzugang (98;100;116) verbunden ist, und
wobei die Substiuat-Kupplung (96;106;112) an einer Bedienungsseite (13) des Hämodialysegerätes (10) angeordnet ist und eine fluidische Schnittstelle zwischen der Internfluidik (12) und der Externfluidik (14) darstellt.

2. Hämodialysegerät (10) nach Anspruch 1, wobei die Substituatpumpe (90;120;132) eine Kolbenpumpe ist.

3. Hämodialysegerät (10) nach einem der vorangegangenen Ansprüche, wobei die Zuleitung (94;102) der Substituatpumpe (90) fluidisch zwischen der Dialysatpumpe (32) und dem Dialysator (50) angeschlossen ist.

4. Hämodialysegerät (10) nach einem der vorangegangenen Ansprüche, wobei die Zuleitung (130) der Substituatpumpe (132) fluidisch zwischen der Dialysatquelle (20) und der Dialysatpumpe (32) angeschlossen ist, und fluidisch parallel zu der Abfallpumpe (34) eine im Parallelstrom betreibbare Ultrafiltrationspumpe (40) vorgesehen ist.

5. Hämodialysegerät (10) nach einem der vorangegangenen Ansprüche, wobei die die Substituatpumpe (90;120;132) eine keramische Pumpe ist.

6. Hämodialysegerät (10) nach einem der Ansprüche 1-5, wobei der Substituatzugang (100) stromabwärts des Dialysators (50) angeordnet ist.

7. Hämodialysegerät (10) nach einem der Ansprüche 1-5, wobei der Substituatzugang (98) stromaufwärts des Dialysators (50) angeordnet ist.

8. Hämodialysegerät (10) nach einem der Ansprüche 1-5, wobei der Substituatzugang (116) an der Blutseite (15) des Dialysators (50) angeordnet ist.

9. Hämodialysegerät (10) nach einem der vorangegangenen Ansprüche, wobei eine zweite mit der Substituatpumpe (120) verbundene Substituatkupplung (106) und eine Ventilanordnung (122) zwischen der Substituatpumpe (106) und den Substituatkupplungen (96;106) angeordnet sind.

10. Hämodialysegerät (10) nach einem der vorangegangenen Ansprüche, wobei der Substituatpumpe (90;120;132) ein Durchfluss-Sensor (92) fluidisch zugeordnet ist.

## Claims

1. Hemodialysis device (10) with permanent and disinfectable internal fluidics (12) inside a device housing (11) and replaceable disposable external fluidics (14) outside the device housing (11), wherein
the external fluidics (14) comprise all blood-side lines (60, 80) and a dialyzer (50), and
the internal fluidics (12) comprise
a balancing pump (30) formed by a dialysate pump (32) and a waste pump (34) coupled with the same, the dialysate pump (32) pumping dialysate from a dialysate source (20) to the dialyzer (50), and the waste pump (34) pumping dialysate from the dialyzer (50) to a waste destination, and
a separate disinfectable substituate pump (90; 120; 132) pumping dialysate from the dialysate source (20) to the external fluidics (14),
wherein the substituate pump (90; 120; 132) is a non-peristaltic displacement pump with a volumetric precision in a single-digit percentage range,
wherein the external fluidics comprise a separate substituate port (98; 100; 116) to the blood side (15) of the external fluidics (14) and a substituate coupling (96; 106; 112) is provided via which the substituate pump (90; 120; 132) is fluidically connected with the substituate port (98; 100; 116), and
wherein the substituate coupling (96; 106; 112) is arranged on an operation side (13) of the hemodialysis device (10) and represents a fluidic interface between the internal fluidics (12) and the external fluidics (14).

2. Hemodialysis device (10) of claim 1, wherein the substituate pump (90; 120; 132) is a piston pump.

3. Hemodialysis device (10) of one of the preceding claims, wherein the feed line (94; 102) of the substituate pump (90; 120; 132) is fluidically connected between the dialysate pump (32) and the dialyzer (50).

4. Hemodialysis device (10) of one of the preceding claims, wherein the feed line (130) of the substituate pump (90; 120; 132) is fluidically connected between the dialysate source (20) and the dialysate pump (32), and wherein an ultrafiltration pump (40) is provided fluidically parallel to the waste pump (34), the ultrafiltration pump being operable in parallel flow.

5. Hemodialysis device (10) of one of the preceding claims, wherein the substituate pump (90; 120; 132) is a ceramic pump.

6. Hemodialysis device (10) of one of claims 1-5, wherein the substituate port (100) is arranged downstream of the dialyzer (50).

7. Hemodialysis device (10) of one of claims 1-5, wherein the substituate port (98) is arranged upstream of the dialyzer (50).

8. Hemodialysis device (10) of one of claims 1-5, wherein the substituate port (116) is arranged on the blood side (15) of the dialyzer (50).

9. Hemodialysis device (10) of one of the preceding claims, wherein a second substituate coupling (106) connected with the substituate pump (120) and a valve arrangement (122) are arranged between the substituate pump (120) and the substituate couplings (96; 106).

10. Hemodialysis device (10) of one of the preceding claims, wherein a flow sensor (92) is fluidically assigned to the substituate pump (90; 120; 132).

## Revendications

1. Appareil d'hémodialyse (10) avec une fluidique interne (12) permanente et désinfectable au sein d'un carter d'appareil (11) et une fluidique externe (14) jetable et remplaçable à l'extérieur du carter d'appareil (11), dans lequel
la fluidique externe (14) comprend tous les conduits (60, 80) côté sang et un dialyseur (50), et
la fluidique interne (12) comporte
une pompe d'équilibrage (30) formée par une pompe à dialysat (32) et une pompe à déchets (34) couplée à celle-ci, la pompe à dialysat (32) pompant du dialysat d'une source de dialysat (20) vers le dialyseur (50) et la pompe à déchets (34) pompant du dialysat du dialyseur (50) vers une destination des déchets, et
une pompe à fluide de substitution (90; 120; 132) séparée et désinfectable, pompant du dialysat de la source de dialysat (20) vers la fluidique externe (14),
la pompe à fluide de substitution (90; 120; 132) est une pompe volumétrique non-péristaltique avec une précision volumétrique dans des pourcentages à un chiffre,
la fluidique externe comprenant une entrée de fluide de substitution (98; 100; 116) séparée au côté sang de la fluidique externe (14), et un raccord de fluide de substitution (96; 106; 112) étant prévu, par lequel la pompe à fluide de substitution (90; 120; 132) est reliée fluidiquement à l'entrée de fluide de substitution (98; 100; 116), et
le raccord de fluide de substitution (96; 106; 112) étant prévu sur une côté d'opération (13) dudit appareil d'hémodialyse (10) et forme un interface fluidique entre la fluidique interne (12) et la fluidique externe (14).

2. Appareil d'hémodialyse (10) selon la revendication 1, dans laquelle la pompe à fluide de substitution (90; 120; 132) est une pompe à piston.

3. Appareil d'hémodialyse (10) selon l'une quelconque des revendications précédentes, dans lequel la conduite d'alimentation (94; 102) de la pompe à fluide de substitution (90) est raccordée fluidiquement entre la pompe à dialysat (32) et le dialyseur (30).

4. Appareil d'hémodialyse (10) selon l'une quelconque des revendications précédentes, dans lequel la conduite d'alimentation (130) de la pompe à fluide de substitution (132) est raccordée fluidiquement entre la source de dialysat (20) et la pompe à dialysat (32), et dans lequel une pompe d'ultrafiltration (40) est prévue fluidiquement en parallèle à la pompe à déchets (34), ladite pompe d'ultrafiltration pouvant être opérée en écoulement parallèle.

5. Appareil d'hémodialyse (10) selon l'une quelconque des revendications précédentes, dans lequel la pompe à fluide de substitution (90; 120; 132) est une pompe en céramique.

6. Appareil d'hémodialyse (10) selon l'une quelconque des revendications 1-5, dans lequel l'entrée de fluide de substitution (100) est située en aval du dialyseur (50).

7. Appareil d'hémodialyse (10) selon l'une quelconque des revendications 1-5, dans lequel l'entrée de fluide de substitution (98) est située en amont du dialyseur (50).

8. Appareil d'hémodialyse (10) selon l'une quelconque des revendications 1-5, dans lequel l'entrée de fluide de substitution (116) est située sur le côté sang (15) du dialyseur (50).

9. Appareil d'hémodialyse (10) selon l'une quelconque des revendications précédentes, dans lequel un deuxième raccord de fluide de substitution (106), qui est lié à la pompe à fluide de substitution (120), et un dispositif de soupape (122) sont agencés entre la pompe à fluide de substitution (120) et les raccords de fluide de substitution (96; 106).

10. Appareil d'hémodialyse (10) selon l'une quelconque des revendications précédentes, dans lequel un capteur de débit (92) est associé fluidiquement à la pompe à fluide de substitution (90; 120; 132).
